# EUROPEAN PATENT SPECIFICATION

(11) **EP 3 310 245 B1**
(45) Date of publication and mention of the grant of the patent: **24.01.2024**
(21) Application number: 16813840.2
(22) Date of filing: 15.06.2016
(51) Int. Cl.: A61B 1/247, A61C 17/02

(54) **NEW DENTAL SUCTION-MIRROR TOOL**
NEUES ZAHNÄRZTLICHES SAUG-SPIEGEL-INSTRUMENT
NOUVEL OUTIL MIROIR DENTAIRE À ASPIRATION

(30) Priority: 22.06.2015 IL 23958215
(43) Date of publication of application: 25.04.2018
(73) Proprietor: Rndent-IP Ltd., 4532915 Hod Hasharon (IL)
(72) Inventor: TAVOR, David, 4532915 Hod Hasharon (IL); AVRAMOV, Nissim, 6428511 Tel Aviv (IL); HAIMOVICH, Roee, 3666003 Nesher (IL)
(74) Representative: Wynne-Jones IP Limited
(86) International application number: PCT/IL2016/050627
(87) International publication number: WO 2016/207876

(56) References cited:
- WO-A1-00/60997
- WO-A1-2014/140795
- FR-A1- 2 595 939
- US-A- 3 829 199
- US-A- 5 230 622
- US-A1- 2010 021 860

## Description

### Field of the Invention

The present invention relates to the field of dental treatment in general, and to a new design of disposable dental mirror tool combined with a dental suction mechanism, in particular.

### Background of the Invention

Various dental mirrors have been used by dentists for many years in order to view inside a patient's mouth. The dental mirrors consist of a small cylindrical metal shaft with a metal disk attached at the end of it, which holds the mirror. Typically, such mirrors are biologically inert, environmentally stable and durable, and are capable of being autoclaved and sterilised, or are disposable.

However, being disposable or not, the dental mirrors have an essential drawback in their design. The reflective surface of the small hand-held dental mirrors become instantly fogged due to moisture and heat in the patient's mouth, or the surface of the mirrors becomes non- reflective due to saliva and debris from drilling operations and other dental procedures.

The conventional method for cleaning the debris formed during the dental procedure is by using a suction tube hold in another hand, which does not hold the mirror, or the hands of an assistant, In addition, the assistant uses cloth to remove fog, saliva and debris from the mirror, or by spraying water on the mirror tool. Such method for cleaning the debris and dental mirror is wasteful in terms of time and effort and makes the dental surgeon's work more cumbersome. Therefore, there is a long- felt need for improvement through the invention of a mechanism or new tool that would simultaneously allow a dentist using only one hand to view in a patient's mouth and effectively clean the debris and the mirror to achieve an operable reflection without having to remove the mirror from the patient's mouth for either cleaning or replacement. This would allow a streamlined, fast and more convenient procedure in a dentist's office for the benefit of both the dentist and the patient. In addition, to increase ergonomics and user-friendliness, such a device should be able to be operated by the dentist in the same hand that normally holds the mirror during dental procedures.

There are some known pre-existing solutions to the problem of designing a dental mirror cleaning device. USD 0320075 shows an instrument for dental care which includes a handle having a mirror at one end and includes provision for enabling suction and water syringe operations via passage from one end to the other end of the instrument.

US 7,553,158 describes a dental evacuation tool suitable to be placed in fluid communication with a dental vacuum source. The tool comprises a suction head and an elongated tubular handle.

US 3,102,338 relates to a dental mirror for use in combination with a fluid-cooled drill which will evacuate fluid from the patient's mouth, which maintains the reflecting surface of the mirror free of the coolant during use.

US 3,986,266 discloses a dental mirror comprising a handle with a reflecting mirror mounted at one end, an air jet tube secured to the handle and arranged to direct a flow of air onto the reflecting face of the mirror, and including a water supply tube from which droplets of water are supplied into the air stream from the air jet tube. WO 2014/140795 discloses a customized mirror with a handle, for medical or dental examination. The mirror has a hydrophobic or super-hydrophobic and non-stick reflecting surface. The mirror includes means for controlling a compressed air flow with an increased downward pressure in an angle of 5 to 25 degrees over the reflecting surface by use of diverging control surfaces.

The above designs and devices are feasible, but lacking in one aspect or another. Some of them require an air and water line simultaneously connected to the mirror hand-piece to allow water and air continuously flow over the mirror surface, removing the opportunity for debris to accumulate. Others require sterilization, and all present various drawbacks. Thus, there is a clear demand for the essential improvement of the current dental mirror tool.

### Summary of the Invention

The present invention relates to a dental suction-mirror tool allowing a dentist simultaneous viewing in the patient's mouth and cleaning debris formed during dental procedures without having to remove the mirror from the mouth for either cleaning or replacement, said tool configured to be manipulated by one hand and characterized by comprising a round or oval mirror frame with an anti-fog mirror head made of a highly reflective thermoplastic polymer, the polymer being glycol-modified polyethylene terephthalate, and handle with a dental suction tube inserted and passed through the handle attached onto said mirror frame at an angle of about 50° relative to the mirror plane, thereby enabling optimal liquid flow and removal of the debris from the mirror, wherein said suction tube and said mirror frame with the mirror constitute a disposable plastic unit. The dental suction tube can be any standard dental suction tube used during dental procedures. It can be either an integral part of the dental suction -mirror tool, such as a disposable continuous plastic piece, or inserted into said tool when needed.

A disposable adhesive and anti-fog mirror can be attached to the mirror frame.

The new dental mirror tool can be used for simultaneously viewing in the patient's mouth and cleaning debris formed during dental procedures.

Various embodiments of the invention may allow various benefits, and may be used in conjunction with various applications. The details of one or more embodiments are set forth in the accompanying figures and the description below. Other features, objects and advantages of the described techniques will be apparent from the description and drawings and from the claims.

### Brief Description of the Drawings

The present invention will be understood and appreciated more fully from the following detailed description taken in conjunction with the appended figures.
Fig. 1 is a perspective view of the dental suction -mirror tool.
Fig. 2 is a top view of the dental suction -mirror tool.
Fig. 3 is a front view of the dental suction -mirror tool.
Fig. 4 is a schematic perspective view of the dental suction -mirror tool with a handle.

### Detailed Description of the Disclosure

In the following description, various aspects of the invention will be described. For purposes of explanation, specific configurations and details are set forth in order to provide a thorough understanding of the invention. However, it will also be apparent to one skilled in the art that the invention may be practiced without the specific details presented herein. Furthermore, well-known features may be omitted or simplified in order not to obscure the invention.

The present invention relates to a dental suction-mirror tool as defined in the claims. The dental suction tube can be any standard dental suction tube used during the dental procedures. It can be either an integral part of the dental suction -mirror tool, or inserted into said tool when needed.

The dental mirror tool of the invention is "hand-held", means suitably sized, weighted and configured to be held in a dentist's hand during use, and to be manipulated by only one hand during the dental procedure. In fact, the dental mirror tool of the invention replaces the pair of tools, namely the mirror and the suction tube, which are used by any dentist today. This makes possible for a dentist to work without employing an assistant, who at the same time might be able to prepare the next step of the treatment independently. That would significantly reduce valuable time of the dental procedures. In addition, simultaneous use of the mirror and suction tool combined in one single instrument allows a dentist cleaning the debris formed during the dental procedure effectively and comfortably for a patient. This is because the dentist, who sees the treated area, knows exactly where and at which angle, the tool should be held and when the suction should be applied.

Thus, it is the object of the present invention to combine the dental mirror and suction tube in a single, one-hand-held instrument to allow a dentist to simultaneously view and clean the treated area without changing dental tools or interrupting the dental procedure.

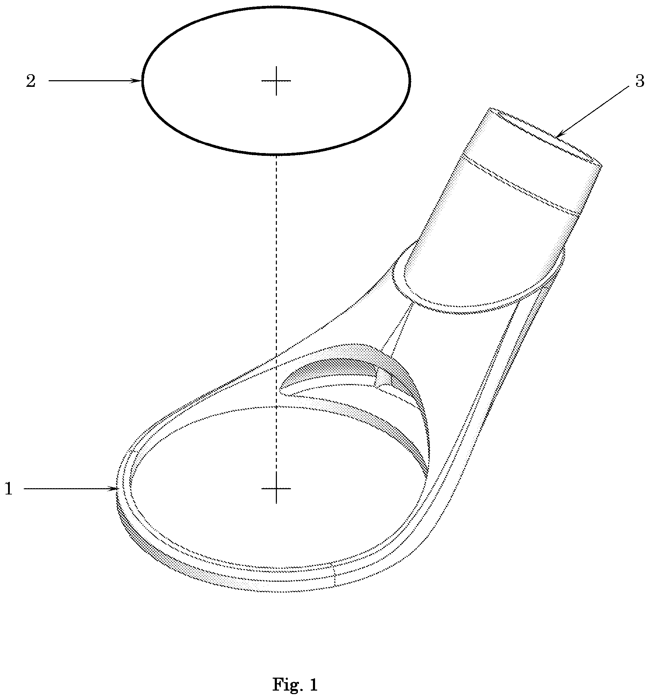

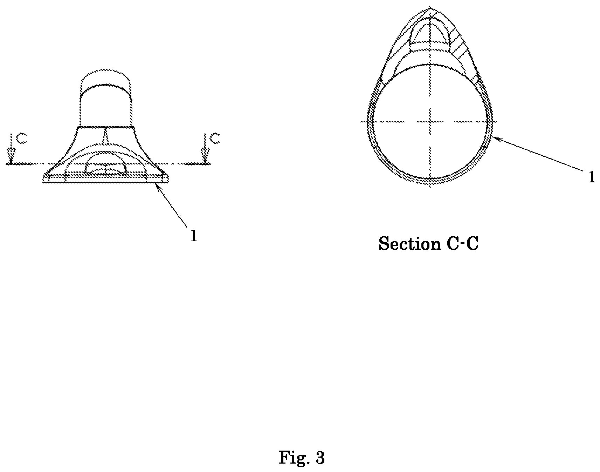

Reference is now made to Figs. 1-3 showing the perspective, top and front views of the new dental suction-mirror tool of the present invention. As shown in Fig. 1, disposable mirror 2 can be attached to mirror frame 1 via the bottom side of the mirror, which is adhesive. Mirror frame 1 is essentially round or oval and has a specifically designed, bent shape for the reasons explained below. Mirror frame 1 forms a direct support structure for disposable mirror 2. Any disposable dental suction tube (not shown), which is available in the dentist's office, can be easily inserted 3 into said mirror frame 1 at an angle of about 50° relative to the mirror plane. Suction tube may serve as a handle for the dental suction -mirror tool.

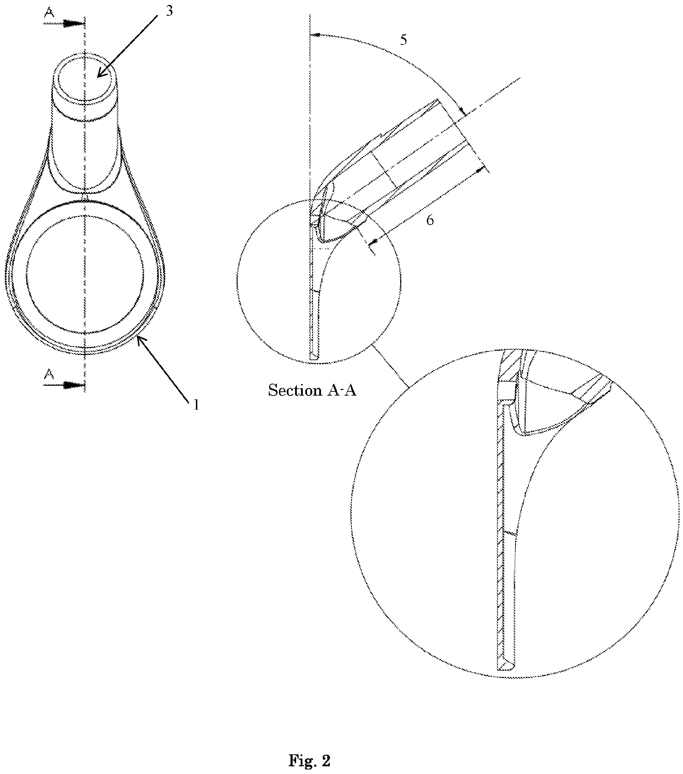

As shown in Fig. 2, due to the very special design of the tool, disposable suction tube can be inserted 3 into mirror frame 1 only at specific angle 5 between the mirror plane and the longitudinal axis of the tube. Rotating the suction tube with the dental suction -mirror tool on it makes it possible to rotate the mirror in the patient's mouth in order to attain the required view. Angle 5 provides a free flow zone 6 for the liquid removed via suction tube, and therefore constitutes one of the major aspects of the present invention. According to the empirical calculations of the flow rate in the free flow zone 6 of the dental mirror tool, optimal liquid flow via suction tube occurs when angle 5 is about 50°, relative to the mirror plane. The term "optimal" means that the debris created during the dental procedure is effectively removed from the mirror 2.

Disposable suction tube has standard size and dimensions as most of the similar suction tubes used in dentistry, and hence, determines the size and dimensions of the mirror head and mirror frame 1. For example, for size 5 disposable suction mirror tool, the diameter of the suction tube is 8 mm. The tube can be easily inserted in the mirror frame and allows an effective suction of the liquid from the mirror, as described above.

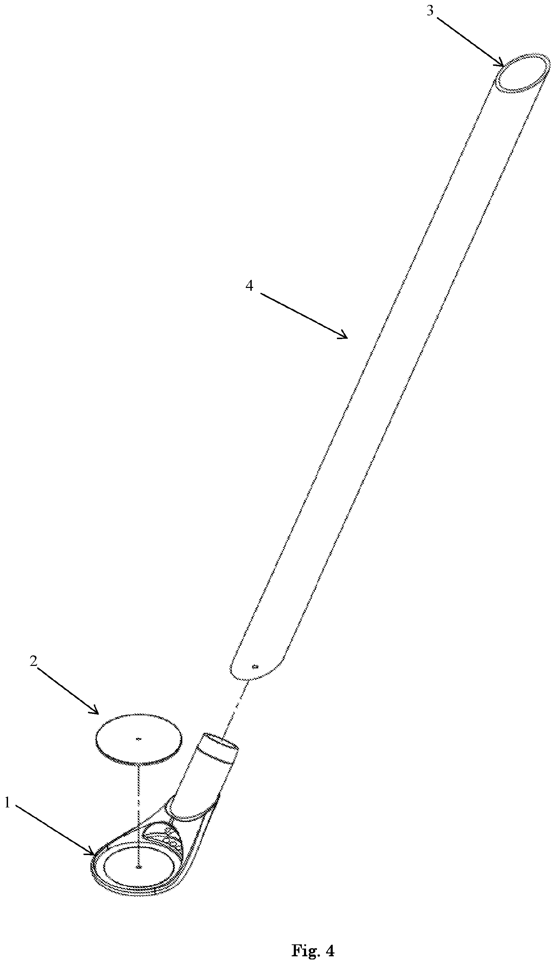

Reference is now made to Fig. 4 showing the dental suction-mirror tool with a handle. This particular dental mirror tool comprises mirror frame 1, disposable mirror 2, optional suction tube (not shown, but inserted 3 inside the handle), and handle 4. Bended mirror frame 1 can be easily attached to, and detached from handle 4. Alternatively, both parts may constitute a disposable single continuous unit or piece of plastic or other material.

Optional handle 4 is used to manipulate the mirror. It may have any suitable geometrical shape and can be straight or curved. The handle may be flexible, smooth or contoured, and should be long enough to easily manipulate the mirror with one hand. A skilled artisan, given the benefits of the disclosure, will be able to select materials, geometries and dimensions for the handle suitable for an intended use. Disposable suction tube is inserted 3 and passed through handle 4. Since mirror frame 1 is bent at angle 5, the suction tube will be positioned at this very specific angle, and the suction will be optimal, as described above.

Disposable mirror 2 is a round or oval sticker having a reflective side and an adhesive side with the attached double-side adhesive tape used to stick the disposable mirror to the mirror frame for use in a dental procedure. The disposable mirror 2 is made of glycol-modified polyethylene terephthalate (PETG), which is a clear and highly reflective thermoplastic polymer. The PETG mirrors are anti-fog, lightweight, biocompatible, flexible, impact resistant and commercially available, for example under trade names Plaskolite^{®} , MirroPlast^{™}, Vivak^{®} and Soectar^{®} . These mirrors have adhesive back with the double-side adhesive tape and custom masking on front and back. One of the main virtues of the PETG products is that they are fully recyclable. Upon completion of the dental procedure, the dental mirror tool can be easily disposed.

Use of the new dental mirror tool allows for simultaneous viewing in the patient's mouth and cleaning debris formed during dental procedures. The new tool can be used virtually in any dentist office or clinics and in any dental environment.

While certain features of the invention have been illustrated and described herein, many modifications, substitutions, changes, and equivalents will be apparent to those of ordinary skill in the art. For example, a dentist can hold more than one tool during the dental procedure. It means that the new dental suction mirror tool can be adapted for use by two hands, by three hands, by four hands or even by an assistant, if needed. It is, therefore, to be understood that the appended claims are intended to cover all such modifications and changes as fall within the scope of the invention.

## Claims

1. A dental suction-mirror tool allowing a dentist simultaneous viewing in the patient's mouth and cleaning debris formed during dental procedures without having to remove the mirror from the mouth for either cleaning or replacement, said tool configured to be manipulated by one hand and comprising a round or oval mirror frame (1) with an anti-fog mirror (2) made of a highly reflective thermoplastic polymer, and a handle (4) with a dental suction tube inserted and passed through the handle (4) attached onto said mirror frame (1) at an angle of about 50° relative to the mirror plane, thereby enabling optimal liquid flow and removal of the debris from the mirror (2), **characterized in that** said suction tube and said mirror frame (1) with the mirror (2) constitute a disposable plastic unit, and **in that** the highly reflective thermoplastic polymer is glycol-modified polyethylene terephthalate.

2. The tool according to claim 1, wherein the mirror (2) is an adhesive anti-fog mirror suitable to be attached to the mirror frame (1).

3. The tool according to claims 1, wherein the dental suction tube is an integral part of the tool, which is a disposable continuous plastic piece.

## Patentansprüche

1. Zahnärztliches Saug-Spiegel-Instrument, das einem Zahnarzt ein gleichzeitiges Betrachten in dem Mund des Patienten und ein Reinigen von Ablagerungen ermöglicht, die während zahnärztlichen Eingriffen gebildet wurden, ohne den Spiegel entweder zum Reinigen oder für einen Austausch aus dem Mund entfernen zu müssen, wobei das Instrument konfiguriert ist, um durch eine Hand manipuliert zu werden und umfassend einen runden oder ovalen Spiegelrahmen (1) mit einem Anti-Beschlagspiegel (2), der aus einem hochreflektierenden thermoplastischen Polymer hergestellt ist,
und einen Griff (4) mit einem zahnärztlichen Saugschlauch, der in den Griff (4) eingeführt und durchgeleitet wird, der in einem Winkel von etwa 50° relativ zu der Spiegelebene an dem Spiegelrahmen (1) angebracht ist, wobei dadurch ein optimaler Flüssigkeitsfluss und eine Entfernung der Ablagerungen von dem Spiegel (2) befähigt werden, **dadurch gekennzeichnet, dass** der Saugschlauch und der Spiegelrahmen (1) mit dem Spiegel (2) eine Einwegkunststoffeinheit ausmachen und **dadurch,** dass das hochreflektierende thermoplastische Polymer Glykol-modifiziertes Polyethylenterephthalat ist.

2. Instrument nach Anspruch 1, wobei der Spiegel (2) ein adhäsiver Anti-Beschlagspiegel ist, der geeignet ist, um an dem Spiegelrahmen (1) angebracht zu werden.

3. Instrument nach Anspruch 1, wobei der zahnärztliche Saugschlauch ein integraler Teil des Instruments ist, das ein ununterbrochenes Einwegkunststoffstück ist.

## Revendications

1. Outil miroir d'aspiration dentaire permettant à un dentiste simultanément de voir dans la bouche du patient et de nettoyer les débris formés pendant des procédures dentaires sans avoir à retirer le miroir de la bouche pour le nettoyer ou le remplacer, ledit outil étant conçu pour être manipulé d'une seule main et comprenant un cadre de miroir rond ou ovale (1) avec un miroir antibuée (2) constitué d'un polymère thermoplastique hautement réfléchissant,
et une poignée (4) avec un tube d'aspiration dentaire inséré et passé à travers la poignée (4) fixée sur ledit cadre de miroir (1) selon un angle d'environ 50° par rapport au plan de miroir, permettant ainsi un écoulement de liquide optimal et l'élimination des débris du miroir (2), **caractérisé en ce que** ledit tube d'aspiration et ledit cadre de miroir (1) avec le miroir (2) constituent une unité en plastique jetable, et **en ce que** le polymère thermoplastique hautement réfléchissant est le polyéthylène téréphtalate modifié au glycol.

2. Outil selon la revendication 1, dans lequel le miroir (2) est un miroir antibuée adhésif adapté pour être fixé sur le cadre de miroir (1).

3. Outil selon la revendication 1, dans lequel le tube d'aspiration dentaire fait partie intégrante de l'outil, qui est une pièce en plastique continue jetable.
